**Europäisches Patentamt**

**European Patent Office**

**Office européen des brevets**

(19)

(11) Veröffentlichungsnummer : **0 182 387**
**B1**

(12) ## EUROPÄISCHE PATENTSCHRIFT

(45) Veröffentlichungstag der Patentschrift :
15.06.88

(51) Int. Cl.⁴ : **A 61 F 5/56**

(21) Anmeldenummer : 85114885.8

(22) Anmeldetag : 23.11.85

(54) **Vorrichtung zur Verhinderung von unartikulierten Geräuschen im Mund- und Rachenraum.**

(30) Priorität : 23.11.84 DE 3442669
23.11.84 DE 3442670

(43) Veröffentlichungstag der Anmeldung :
28.05.86 Patentblatt 86/22

(45) Bekanntmachung des Hinweises auf die Patenterteilung : 15.06.88 Patentblatt 88/24

(84) Benannte Vertragsstaaten :
**AT BE CH DE FR GB IT LI LU NL SE**

(56) Entgegenhaltungen :
**DE-C- 407 949**
**DE-C- 656 806**
**US-A- 1 674 336**
**US-A- 3 132 647**

(73) Patentinhaber : Pecanov, Atanas, Dr. med. dent.
Remigiustrasse 78
D-4060 Viersen 1 (DE)

(72) Erfinder : Pecanov, Atanas, Dr. med. dent.
Remigiustrasse 78
D-4060 Viersen 1 (DE)

(74) Vertreter : Uhlmann, Hans, Dr. rer.nat., Dipl.-Chem.
Gladbacher Strasse 189
D-4060 Viersen 1 (DE)

EP 0 182 387 B1

## Beschreibung

Die Erfindung betrifft eine Vorrichtung zur Verhinderung von unartikulierten Geräuschen im Mund- und Rachenraum, bestehend aus einem aus zwei Schenkeln bestehenden Aufbißteil zur Befestigung der Vorrichtung am Oberkiefer und einem mit der Zunge in Eingriff stehenden Eingriffsteil, durch das die Zunge im Abstand zum Gaumen des Oberkiefers gehalten wird.

Die Entstehung unartikulierter Geräusche im Mund- und Rachenraum, im Volksmund und im weiteren Beschreibungstext kurz mit Schnarchen bezeichnet, stellt ein soziales Phänomen dar, insbesondere dort, wo Menschen in einem Raum zusammen schlafen müssen, wie in Schlafzimmern von Ehepaaren, Hotels, in Kasernen und im Schlafwagen. Dabei ist der störende Einfluß des Schnarchens auf den anderen Partner bzw. die Mitbewohner häufig derart gravierend, daß Ehen gefährdet sind oder in Kasernen, Wohnheimen und Altersheimen Aggressivität die Folge ist.

Die Medizin hat sich deshalb seit langem dieses Problems angenommen und versucht, durch operative Eingriffe das Schnarchen zu verhindern oder zu reduzieren, beispielsweise durch die Entfernung der Mandeln und weicher Teile des Gaumens. Abgesehen davon, daß diese operativen Eingriffe unangenehm sind, sind sie auch gesundheitlich nicht unbedenklich, da sie anatomische Veränderungen im Mund- und Rachenraum schaffen, die während des Schlafes zwar die positive Auswirkung des verminderten Schnarchens haben können, tagsüber aber die Region des Mund- und Rachenraums gefährden und den Eintritt von Krankheitskeimen, kalter Luft u. ä. ermöglichen.

Es sind deshalb schon eine Reihe von Vorschlägen gemacht worden, durch Einbringen von Vorrichtungen im Mund- und Rachenraum das Schnarchen zu verhindern, ohne daß bisher das Problem befriedigend und in für den Betroffenen erträglicher Weise gelöst ist. So sieht die DE-PS 407 949 und die US-PS 31 32 647 eine Gaumenplatte vor, die der Anatomie des Oberkiefers genau angepaßt ist und an diesem eng anliegt und an deren hinterem Ende ein Bügel angebracht ist, der nach hinten in den Rachenraum hineinragt und die Zungenwurzel nach unten drückt und dadurch einen Abstand zwischen Zungenwurzel und weichem Gaumen schafft. Dadurch kann die Luft verhältnismäßig frei austreten und die gefürchteten Schnarchgeräusche können vermieden oder zumindest vermindert werden. Bei der Vorrichtung nach der US-PS 31 32 647 ist an dem in den Rachenraum hineinragenden Bügel entweder eine Platte oder aber für Patienten mit sehr schlaffem weichem Gaumen ein rohrförmiger Körper angebracht, der Zungenwurzel und weichen Gaumen trennt und durch den die Luft hindurchtreten kann.

Daß diese in den beiden Schriften gemachten Vorschläge keine Verbreitung gefunden und das dringend anstehende Problem nicht gelöst haben, ist insbesondere darin begründet, daß jede im Mund einzubringende Vorrichtung einen Fremdkörper darstellt und für den Benutzer unangenehm ist. Bei den beiden vorbekannten Vorrichtungen trifft das in besonders starkem Maße zu, weil der Bügel oder Tragarm ausgerechnet in den Teil des Mund- und Rachenraumes hineinragt, bei dem ein Fremdkörper stets zum Brechreiz führt. Außerdem ist auch die vollständige bzw. fast vollständige Abdeckung des oberen Gaumens unangenehm.

Der Erfindung liegt deshalb die Aufgabe zugrunde, eine Vorrichtung zu schaffen, die diese Nachteile nicht aufweist, zu keinem Brechreiz führt und möglichst große Bereiche des Gaumens frei hält von einem Fremdkörper.

Diese Aufgabe wird bei einer Vorrichtung zur Verhinderung von unartikulierten Geräuschen im Mund- und Rachenraum, die aus einem aus zwei Schenkeln bestehenden Aufbißteil zur Befestigung der Vorrichtung am Oberkiefer und einem mit der Zunge in Eingriff kommenden Eingriffsteil besteht, dadurch gelöst, daß das Eingriffsteil aus einem mittig zwischen den Schenkeln des Aufbißteils angeordneten Gaumenteil besteht, das dazu bestimmt ist, sowohl mit der Mitte des harten Gaumens als auch mit der Mitte des Zungenkörpers (dorsum linguae) im Eingriff zu stehen.

Der Vorteil der erfindungsgemäßen Vorrichtung besteht darin, daß das Gaumenteil, durch das sichergestellt wird, daß Gaumen und Zunge voneinander getrennt bleiben und damit Schnarchgeräusche vermieden werden, nicht in den Bereich des weichen Gaumens und der Zungenwurzel hineinragt und dort zu einem unvermeidlichen Brechreiz führt. Die Anordnung des Gaumenteils zwischen den Schenkeln des Aufbißteils, d. h. als zentrale, kompakte Einheit, hat weiter den Vorteil, daß sie zwar sicher Gaumen und Zunge im Abstand zueinander hält, aber bis auf den Mittelteil sowohl den Gaumen als auch die Zunge frei und damit unbeeinflußt läßt und daß durch diese zentrale Anordnung sehr viel Freiraum geschaffen wird, durch den die Luft ungehindert hindurchströmen kann.

Die Verbindung zwischen dem Gaumenteil und dem der Befestigung der Vorrichtung im Mund dienenden Aufbißteil zu einer leicht einzubringenden Einheit erfolgt zweckmäßig über Drähte, die durch die einzelnen untereinander zu verbindenden Teile hindurchgeführt sind.

Bei einer besonders vorteilhaften Ausführungsform nach der Erfindung für Benutzer, die im Oberkiefer noch weitgehend ihre natürlichen Zähne haben, besteht die im Oberkiefer zu befestigende Vorrichtung aus einem frontalen Umfassungsteil zur Umfassung des processus alvolearis und einem Aufbißteil mit zwei voneinander getrennten Schenkeln zur Auflage auf den Backenzahnen und einem Gaumenteil. Alle diese Teile sind mittels Drähten untereinander verbunden, so daß sich eine einstückige, einfach in den Mundraum einzubringende und tagsüber wieder zu entfernende Vorrichtung ergibt. Als Materialien für das Umfassungsteil, das Aufbißteil und das Gaumenteil kommen eine Vielzahl von Stoffen in Betracht wie Metall, Porzellanmassen oder Schaumglas. Bevorzugt sind jedoch Kunststoffe, zweckmäßig physiologisch verträgliche Kunststoffe, wie sie für dentale Prothesen bekannt sind. Besonders bewährt haben sich dafür bestimmte Polyacrylate.

Vorzugsweise erstreckt sich das Umfassungsteil, mit dem die Vorrichtung den processus alveolaris umfaßt, bis zum Bereich der Eckzähne. Die Ausbildung des Befestigungsteils als frontales Umfassungsteil und seitlichen, nur den Bereich der Backenzähne übergreifenden Schenkeln des Aufbißteils hat den Vorteil, daß der überwiegende Teil der empfindlichen Schleimhäute frei bleibt, aber trotzdem ein sehr sicherer Sitz der Vorrichtung gewährleistet ist.

Das Gaumenteil ist in einer bevorzugten Ausführungsform in ein mit dem Gaumen in Eingriff kommendes Oberteil und ein mit der Zunge in Eingriff kommendes Unterteil unterteilt und diese beiden Teile sind bevorzugt in vertikaler Richtung im Abstand zueinander veränderbar. Dabei wird diese Abstandsveränderung bevorzugt über Dehnschrauben bewirkt, die das Oberteil und Unterteil miteinander verbinden. Solche Dehnschrauben geben dem Träger der Vorrichtung die Möglichkeit, mit einem einfachen Werkzeug, beispielsweise einem Metallstift, durch Eingriff in eine Ausnehmung in der Dehnschraube und seitliche Bewegung den Abstand von Unterteil und Oberteil auf die optimale Wirkung zur Unterdrückung des Schnarchens einzustellen. Solche Dehnschrauben sind von der Kieferregulierung her bekannt und bestehen aus einer mittleren, die Dehnung bewirkenden Mittelschraube aus Gewindehülse und Gewindestift und je einem seitlichen Pfosten, bei dem ein Stiftteil in einem Hülsenteil gleitend gelagert ist.

Zweckmäßig sind Ober- und Unterteil des Gaumenteils auch noch in horizontaler Richtung verschiebbar, so daß insbesondere der Eingriffsort mit der Zunge — je nach den Gegebenheiten des Benutzers — variiert und etwas mehr nach vorn oder hinten verlagert werden kann. Das kann zweckmäßig dadurch geschehen, daß Ober- und Unterteil auf Schlitten oder Schienen in horizontaler Richtung gegeneinander nach vorn oder hinten bewegt werden können.

Bei der Ausführungsform des Befestigungsteils als Umfassungsteil und Aufbißteil erfolgt die Verbindung des Umfassungsteils, der Schenkel des Aufbißteils und des Gaumenteils zweckmäßig mittels zweier Drähte, die im Umfassungsteil horizontal und parallel zueinander verlaufen, sich daran anschließend in der Weise verzweigen, daß der obere der beiden Drähte über den wesentlichen Bereich der Wurzeln der Backenzähne in horizontaler Richtung frei weiter verläuft und erst am hinteren Ende der Schenkel des Aufbißteils nach unten abgebogen und mit diesen verbunden ist, wäßhrend der untere der beiden Drähte unmittelbar nach Austritt aus dem Umfassungsteil nach unten zu den vorderen Enden der Schenkel des Aufbißteils abgebogen und mit deren vorderen Enden verbunden ist. Der frei verlaufende obere Draht dient dabei zur Führung der Vorrichtung im Bereich der Wurzeln der Backenzähne.

Für Träger von Zahnvollprothesen im Oberkiefer wird die Vorrichtung zweckmäßig in der Weise gestaltet, daß das zur Befestigung dienende Aufbißteil in seiner Form der Oberkiefer-Zahnvollprothese entspricht. Für den Benutzer hat diese Ausführungsform den Vorteil, daß er beim Schlafengehen lediglich seine Zahnvollprothese austauscht gegen eine gänzlich gleich gestaltete Vorrichtung zum Verhindern des Schnarchens, die als solche lediglich zusätzlich noch das Gaumenteil aufweist. Auch bei dieser Ausführungsform für Prothesenträger sind Gaumenteil und Aufbißteil wieder über Drähte miteinander verbunden. Alle Vorteile der erfindungsgemäßen Vorrichtung, wie sie bei der Ausführung für Benutzer mit natürlichen Zähnen beschrieben sind, bleiben auch bei der Ausführungsform für Prothesenträger voll erhalten.

Die Herstellung der Vorrichtung in beiden Ausführungsformen erfolgt ähnlich wie bei der Herstellung einer Zahnprothese durch den Zahnarzt in Verbindung mit einem Zahntechniker in der Weise, daß zunächst ein Abdruck des Oberkiefers genommen wird. Von diesem Abdruck wird ein Gipsmodell gefertigt, nach dem dann die Kunststoffteile gefertigt werden.

Die in der Beschreibung gewählten Begriffe obere, untere, vordere und hintere beziehen sich auf die Lage der Teile der Vorrichtung, wie sie nach der Befestigung am Oberkiefer vorliegt.

Die Erfindung wird nachstehend anhand von sechs Zeichnungsskizzen näher beschrieben.

Figur 1 zeigt die Draufsicht auf die Vorrichtung, eingesetzt in ein Gipsmodell des Oberkiefers, wobei der einfacheren Darstellung wegen das Gipsmodell um 180 Grad gedreht und damit in dieser Zeichnung unten und oben vertauscht sind,

Figur 2 die Vorrichtung von vorn leicht schräg nach oben gekippt,

Figur 3 die Ansicht von hinten, ebenfalls leicht schräg nach oben gekippt,

Figur 4 die Vorrichtung in Seitenansicht, wobei lediglich einer der Schenkel des Aufbißteils dargestellt, das andere der besseren Übersichtlichkeit wegen aber weggelassen ist,

Figur 5 die Vorrichtung für Träger von Oberkiefer-Zahnvollprothesen, wobei auch in diesem Fall die Vorrichtung um 180 Grad gedreht und damit unten und oben vertauscht sind,

Figur 6 die Seitenansicht des Gaumenteils 3.

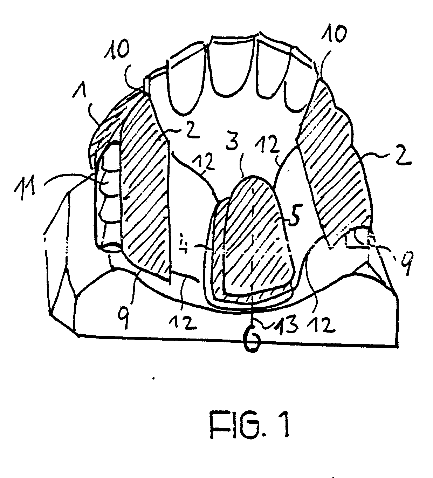

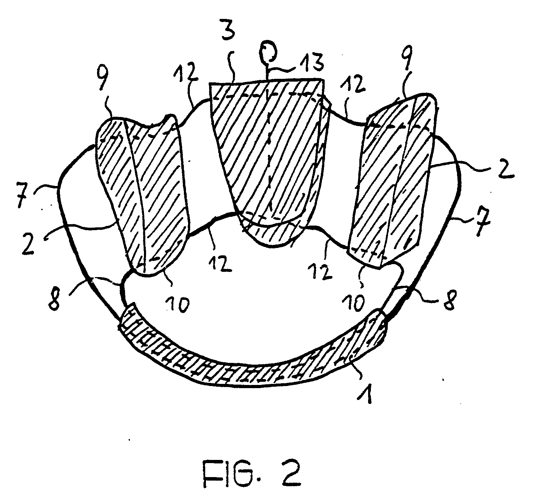

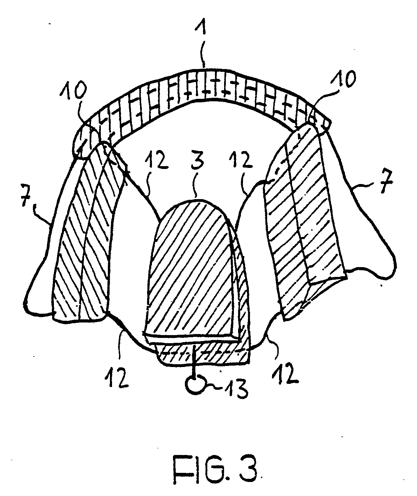

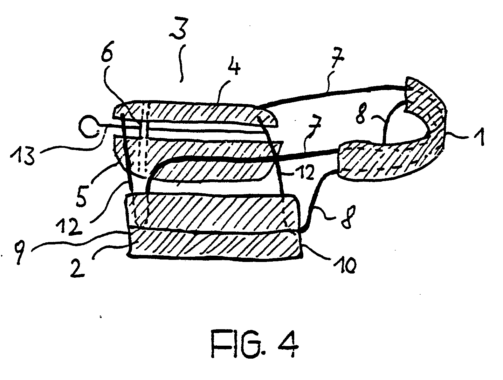

Bei der Vorrichtung gemäß den Figuren 1 bis 4 und 6 ist das aus Kunststoff bestehende Umfassungsteil (1) über den oberen Verbindungsdraht (7) mit den hinteren Enden (9) der beiden Schenkel des Aufbißteils (2), die ebenfalls aus Kunststoff bestehen, verbunden. Über den unteren, nach unten abgebogenen Verbindungsdraht (8) erfolgt die Verbindung mit den vorderen Enden (10) der beiden Schenkel des Aufbißteils (2). Die Verbindungsdrähte (7, 8) sind in die Aufbißteile (2) und das Umfassungsteil (1) eingelagert und durch diese hindurchgeführt. Die beiden Schenkel des Aufbißteils (2) haben eine durchschnittliche Stärke von ca. 3 mm und sind zur Auflage auf den Backenzähnen (11) bestimmt. Die oberen und unteren Verbindungsdrähte (7, 8) führen von den beiden Schenkeln des Aufbißteiles (2) über Drahtstücke (12) nach oben zum ebenfalls aus Kunststoff bestehenden Gaumenteil (3) und verbinden so die Schenkel des Aufbißteils (2) und das Gaumenteil (3) und indirekt damit auch das Umfassungsteil (1) untereinander, so daß sich eine einheitliche, leicht im Mundraum einzubringende Vorrichtung ergibt.

Das Gaumenteil (3) ist ungefähr 2,5 cm lang und 1,5 cm breit und 2 cm hoch und besteht aus dem mit dem Gaumen in Eingriff kommenden Oberteil (4), das der Gaumenform angepaßt ist und in seiner Oberfläche nur ganz leicht gewölbt ist, und dem Unterteil (5), das wesentlich stärker gewölbt ist und sich so etwas in die Zunge eindrücken und diese damit im Abstand zum Gaumen halten kann, ohne daß das Gaumenteil (3) als störend empfunden wird.

Oberteil (4) und Unterteil (5) sind durch die in beide Teile eingebettete Dehnschraube (6) miteinander verbunden und über diese in ihrem Abstand zueinander veränderbar. Das wird bewirkt durch Rechts- oder Linksdrehung der Dehnschraube (6) mittels des Metallstiftes (13), der in eine nicht dargestellte Ausnehmung in der Dehnschraube (6) eingreift. Die Dehnschraube (6) ist von der Kieferregulierung her bekannt und deshalb nicht in Einzelheiten dargestellt. Sie besteht aus einer mittleren, die Dehnung bewirkenden Mittelschraube aus Gewindehülse und Gewindestift und je einem seitlichen Pfosten, bei dem ein Stiftteil in einem Hülsenteil gleitend gelagert ist.

Aufbißteil (2), Gaumenteil (3) und Umfassungsteil (1) bestehen aus einem hautfarben eingefärbten Kunststoff auf Basis Polyacrylat. Alle Kunststoffteile sind an ihren Rändern abgerundet, um Verletzungen bzw. Reizungen der Schleimhäute und der Zunge zu vermeiden.

Die Verbindungsdrähte (7, 8) sind vorzugsweise aus einer Edelmetallegierung, wie einer Chrom-Kobalt-Molybdänlegierung hergestellt, damit keine Korrosion im Mundbereich stattfindet.

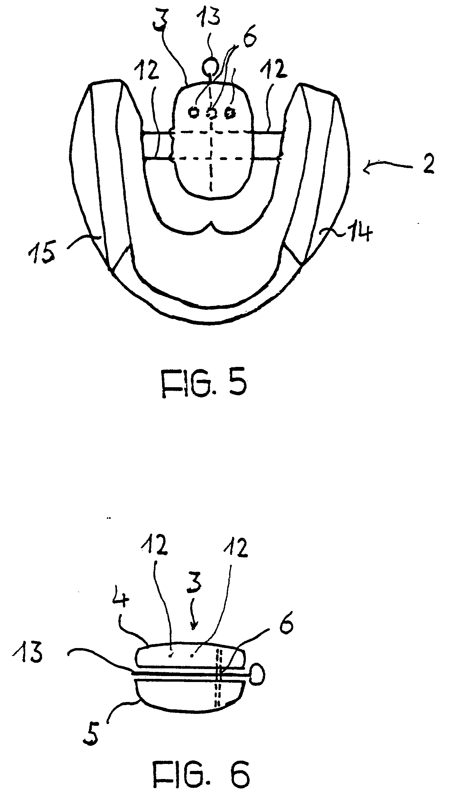

Die Vorrichtung gemäß Figur 5 ist für Träger von Oberkiefer-Zahnvollprothesen bestimmt. Soweit die einzelnen Teile in ihren Funktionen mit denen der Vorrichtung solcher Benutzer übereinstimmt, die noch ihre natürlichen Zähne haben, sind gleiche Bezugszeichen gewählt worden.

Aufbißteil (2) aus Polyacrylat-Kunststoff entspricht in Form und Gestalt im wesentlichen den ursprünglichen Zähnen und dem Oberkiefer des Trägers bzw. seiner entsprechenden Zahnvollprothese. Die beiden Schenkel des Aufbißteils (2) sind bei dieser Ausführungsform Teil eines einzigen zusammenhängenden Kunststoffteiles. Das Aufbißteil (2) ist über die beiden Drahtstücke (12) aus einer Chrom-Kobalt-Molybdänlegierung mit dem Gaumenteil (3) verbunden, wobei die Drahtstücke (12) durch das Oberteil (4) hindurchgehen und jeweils an ihren beiden Enden ca. 0,5 bis 1 cm in den Schenkeln (14, 15) des Aufbißteils (2) verankert sind.

## Patentansprüche

1. Vorrichtung zur Verhinderung von unartikulierten Geräuschen im Mund- und Rachenraum, bestehend aus einem aus zwei Schenkeln bestehenden Aufbißteil (2) zur Befestigung der Vorrichtung am Oberkiefer und einem mit der Zunge in Eingriff kommenden Eingriffsteil, durch das die Zunge im Abstand zum Gaumen des Oberkiefers gehalten wird, dadurch gekennzeichnet, daß das Eingriffsteil aus einem mittig zwischen den Schenkeln angeordneten Gaumenteil (3) besteht, welches dazu bestimmt ist, sowohl mit der Mitte des harten Gaumens als auch mit der Mitte des Zungenkörpers (dorsum linguae) im Eingriff zu stehen.

2. Vorrichtung nach Anspruch 1, dadurch gekennzeichnet, daß das Gaumenteil (3) in ein mit dem Gaumen in Eingriff kommendes Oberteil (4) und ein mit der Zunge in Eingriff kommendes Unterteil (5) unterteilt ist.

3. Vorrichtung nach Anspruch 2, dadurch gekennzeichnet, daß das Oberteil (4) und das Unterteil (5) in vertikaler Richtung im Abstand zueinander veränderbar sind.

4. Vorrichtung nach einem der Ansprüche 2 oder 3, dadurch gekennzeichnet, daß das Oberteil (4) und das Unterteil (5) über Dehnschrauben miteinander verbunden sind.

5. Vorrichtung nach einem der Ansprüche 2 bis 4, dadurch gekennzeichnet, daß das Oberteil (4) und das Unterteil (5) in horizontaler Richtung im Abstand zueinander veränderbar sind.

6. Vorrichtung nach einem der Ansprüche 1 bis 5, dadurch gekennzeichnet, daß das Gaumenteil (3) über Drähte (7, 8) mit dem Aufbißteil (2) verbunden ist.

7. Vorrichtung nach einem der Ansprüche 1 bis 6, dadurch gekennzeichnet, daß für Benutzer mit natürlichen Zähnen im oberen Kiefer zur Befestigung der Vorrichtung das Aufbißteil (2) als Auflageteil zur Auflage auf den Backenzähnen ausgebildet ist und ein frontales Umfassungsteil (1) zur Umfassung des processus alveolaris zwischen beiden Eckzähnen vorhanden ist und Umfassungsteil (1), Aufbißteil (2) und Gaumenteil (3) durch Drähte (7, 8) untereinander verbunden sind.

8. Vorrichtung nach Anspruch 7, dadurch gekennzeichnet, daß die Verbindung des Umfassungsteils (1), der Schenkel des Aufbißteils (2) und des Gaumenteils (3) mittels der Drähte (7, 8) derart erfolgt, daß die Drähte (7, 8) im Umfassungsteil (1) horizontal und parallel zueinander verlaufen, sich daran anschließend in der Weise verzweigen, daß der obere (7) der beiden Drähte (7, 8) über den wesentlichen Bereich der Wurzeln der Backenzähne in horizontaler Richtung weiter verläuft und an den hinteren Enden (9) der Schenkel des Aufbißteils (2) nach unten abgebogen und mit den hinteren Enden (9) verbunden ist, der untere (8) der beiden Drähte (7, 8) unmittelbar nach Austritt aus dem Umfassungsteil (1) nach unten zu den vorderen Enden (10) der Schenkel des Aufbißteils (2) abgebogen und mit deren vorderen Enden (10) verbunden ist.

9. Vorrichtung nach einem der Ansprüche 1 bis 6 für Träger von Oberkiefer-Zahnvollprothesen, dadurch gekennzeichnet, daß das Aufbißteil (1) in seiner Form der Oberkiefer-Zahnvollprothese entspricht.

## Claims

1. Apparatus for preventing unarticulated sounds in the buccal cavity and the pharynx, consisting of a bite portion (2), which comprises two legs, for securing the apparatus to the upper jaw and an engaging portion that engages the tongue and keeps the tongue at a distance from the palate of the upper jaw, characterised in that the engaging portion consists of a palate portion (3), arranged centrally between the legs, which is intended to engage both the centre of the hard palate and the centre of the upper surface of the tongue (dorsum linguae).

2. Apparatus according to claim 1, characterised in that the palate portion (3) is divided into an upper portion (4) that engages the palate and a lower portion (5) that engages the tongue.

3. Apparatus according to claim 2, characterised in that the separation of the upper portion (4) and the lower portion (5) in a vertical direction can be adjusted.

4. Apparatus according to claim 2 or claim 3, characterised in that the upper portion (4) and the lower portion (5) are connected to one another by expanding screws.

5. Apparatus according to any one of claims 2 to 4, characterised in that the separation of the upper portion (4) and the lower portion (5) in a horizontal direction can be adjusted.

6. Apparatus according to any one of claims 1 to 5, characterised in that the palate portion (3) is connected by wires (7, 8) to the bite portion (2).

7. Apparatus according to any one of claims 1 to 6, characterised in that for securing the apparatus in users with natural teeth in the upper jaw the bite portion (2) is designed as a supporting portion for placing on the molars and a frontal encompassing portion (1) for encompassing the processus alveolaris is provided between the two canine teeth and the encompassing portion (1), the bite portion (2) and the palate portion (3) are connected to one another by wires (7, 8).

8. Apparatus according to claim 7, characterised in that the encompassing portion (1), the legs of the bite portion (2) and the palate portion (3) are connected by means of the wires (7, 8) in such a manner that in the encompassing portion (1) the wires (7, 8) are horizontal and parallel to one another and thereafter branch in such a manner that the upper wire (7) of the two wires (7, 8) continues over the main region of the roots of the molars in a horizontal direction and at the back ends (9) of the legs of the bite portion (2) is bent downwards and connected to the back ends (9) and the lower wire (8) of the two wires (7, 8) is bent downwards to the front ends (10) of the legs of the bite portion (2) immediately after leaving the encompassing portion (1) and is connected to the front ends (10).

9. Apparatus according to any one of claims 1 to 6 for wearers of full upper dental prostheses, characterised in that the bite portion (1) corresponds in shape to the full upper dental prosthesis.

## Revendications

1. Dispositif pour éviter la production de bruits inarticulés dans la cavité pharyngobuccale, constitué par une partie de morsure (2) faite de deux branches pour fixer le dispositif à la mâchoire supérieure, et d'une partie de contact venant au contact de la langue qu'elle maintient à distance du palais, caractérisé par le fait que la partie de contact est constituée par une partie palais (3) agencée en position médiane entre les branches, destinée à être en contact aussi bien avec le milieu du voile du palais qu'avec le milieu de la langue (dorsum linguae).

2. Dispositif selon revendication 1, caractérisé par le fait que la partie de contact (3) est subdivisée en une partie supérieure (4) venant au contact du palais et une partie inférieure (5) venant au contact de la langue.

3. Dispositif selon revendication 2, caractérisé par le fait que la partie supérieure (4) et la partie inférieure (5) ont un écartement mutuel variable en direction verticale.

4. Dispositif selon l'une des revendications 2 ou 3, caractérisé par le fait que la partie supérieure (4) et la partie inférieure (5) sont reliées l'une à l'autre par des vis d'écartement.

5. Dispositif selon l'une des revendications 2 à 4, caractérisé par le fait que la partie supérieure (4) et la partie inférieure (5) ont un écartement mutuel variable en direction horizontale.

6. Dispositif selon l'une des revendications 1 à 5, caractérisé par le fait que la partie palais (3) est assemblée à la partie de morsure (2) par des fils (7, 8).

7. Dispositif selon l'une des revendications 1 à 6, caractérisé par le fait que, pour fixer le dispositif dans le cas où l'utilisateur a des dents naturelles à sa mâchoire supérieure, la partie de morsure (2) est réalisée en tant que partie d'appui pour prendre assise sur les molaires, et une partie enveloppante (1) est prévue pour envelopper le processus alveolaris entre les deux canines, la partie enveloppante (1), la partie de morsure (2) et la partie palais (3) étant assemblées les unes aux autres par des fils (7, 8).

8. Dispositif selon revendication 7, caractérisé par le fait que l'assemblage de la partie enveloppante (1), des branches de la partie de morsure (2) et de la partie palais (3) au moyen des fils (7, 8) est réalisé de façon que les fils (7, 8) suivent dans la partie enveloppante (1) un trajet horizontal en étant parallèles l'un à l'autre et divergent ensuite de manière que celui des deux fils (7, 8) qui est le fil supérieur (7) se prolonge en direction horizontale, sur la majeure partie des racines des molaires, et, aux extrémités postérieures (9) des branches de la partie de morsure (2), soit coudé vers le bas et assemblé aux extrémités postérieures (9), et que celui des deux fils (7, 8) qui est le fil inférieur (8) soit, immédiatement après sa sortie de la partie enveloppante (1), coudé vers le bas en direction des extrémités antérieures (10) des branches de la partie de morsure (2) et assemblé auxdites extrémités antérieures (10).

9. Dispositif selon l'une des revendications 1 à 6, pour porteurs de prothèses dentaires totales de maxillaire supérieur, caractérisé par le fait que la partie de morsure (2) correspond, quant à sa forme, à la prothèse dentaire totale de maxillaire supérieur.

FIG. 1

FIG. 2

FIG. 3

FIG. 4

FIG. 5

FIG. 6